# EUROPEAN PATENT SPECIFICATION

(11) **EP 2 100 582 B1**
(45) Date of publication and mention of the grant of the patent: **28.11.2012**
(21) Application number: 08152513.1
(22) Date of filing: 10.03.2008
(51) Int. Cl.: A61K 8/06, A61K 8/19, A61K 8/26, A61K 8/29, A61K 8/35, A61K 8/891, A61K 8/92, A61Q 1/02, A61Q 17/04

(54) **Cosmetic composition**
Kosmetische Zusammensetzung
Composition cosmétique

(43) Date of publication of application: 16.09.2009
(73) Proprietor: The Procter & Gamble Company, Cincinnati, OH 45202 (US)
(72) Inventor: Jones, Neil John, Staines, Middlesex TW18 1ET (GB)
(74) Representative: Simpson, Kirsty Mairi

(56) References cited:
- EP-A- 1 310 239
- WO-A-00/15180
- WO-A-02/22087
- WO-A-2005/102248
- WO-A-2007/011432
- JP-A- 2004 256 471
- US-A- 5 547 659

## Description

### FIELD OF THE INVENTION

According to a first aspect, the present invention relates to a cosmetic composition comprising a continuous aqueous phase, a first dispersed, non-aqueous phase comprising a hydrophobic colorant, and a second dispersed, non-aqueous phase being substantially free of a hydrophobic colorant. According to further aspects, the present invention relates to a process for preparing this composition and the use of this composition to provide coloration to the skin.

### BACKGROUND OF THE INVENTION

Cosmetic compositions may be used to provide different benefits and to meet different consumers' and users' needs. Depending on the benefits expected, a wide range of cosmetic compositions is available to consumers and/or users.
Cosmetics, including make-ups and foundations, may be used to provide aesthetic benefits to skin, e.g. instant coloration of skin and coverage of skin irregularities. These compositions, suitable to provide coloration and coverage to the skin, may comprise colorants, particularly metal oxide pigments such as iron oxide pigments and titanium dioxide pigments.
Skin care compositions may be used to prevent and/or regulate and/or treat skin's conditions and/or protect skin against environmental factors. Particularly, these compositions may provide acute and/or chronic care benefits to skin, e.g. moisturizing, self-tanning, anti-aging and UV-protecting benefits. They may comprise various skin care actives, e.g. vitamin compounds, anti-aging actives, humectants, self-tanning compounds, and UV-sunscreens compounds.
Nonetheless, there is a constant need for providing one cosmetic composition suitable to meet several consumers' and users' needs. Particularly, there is an increasing need for providing compositions providing both aesthetic benefits and skin care benefits. However, the incorporation of several different types of components may be detrimental to the aesthetics of the composition. This may also impair the application experience, e.g. by increasing the stickiness of the composition. This incorporation may also impair the stability of the composition as some components would be non-compatible to each other. There is a need, therefore, for providing a composition providing both aesthetic benefits and skin care benefits while having satisfactory aesthetics. There is also a need for providing a composition providing both aesthetic benefits and skin care benefits without compromising the application experience. Finally, there is a need for providing a composition providing both aesthetic benefits and skin care benefits while being stable over a sufficient period of time. In other words, there is a need for providing a composition comprising components being non-compatible to each other without compromising its stability.
In addition, consumers usually associate the appearance of a composition with specific benefits. A composition having a dark color, e.g. brown or black, is usually considered as providing aesthetic benefits. A composition having a light color, e.g. white or beige, is usually considered as providing skin care benefits, particularly moisturizing benefits. In contrast, the consumers tend to underestimate the benefits effectively provided by the composition when the composition appearance mismatches the benefits expected, e.g. a moisturizing composition having a dark color. Consequently, there is a need for providing a composition having an appearance which matches both the aesthetic benefits and the skin care benefits it provides to the skin.
Finally, there is a constant need for providing a composition providing a superior application experience. Particularly, there is a need for a composition where the delivery of a component, upon application of the composition to the skin, is associated with the modification of the appearance of the composition. Preferably, there is a need for a composition where the delivery of a component, upon application of the composition to the skin, is associated with the modification of the coloration of the composition

### SUMMARY OF THE INVENTION

According to a first aspect, the present invention relates to a cosmetic composition comprising:
(1) a continuous aqueous phase;
(2) a first dispersed, non-aqueous phase being solid at room temperature, having a melting point from 27°C to 60°C, and comprising a hydrophobic colorant; and,
(3) a second dispersed, non-aqueous phase being distinct from the first non-aqueous phase, and being substantially free of a hydrophobic colorant;
   wherein the second non aqueous phase comprises at least are lipophilic component, which component being non-compatible with hydrophobic colorants

According to a second aspect, the present invention relates to a process for preparing a cosmetic composition, according to the first aspect, this process comprising the steps of:
(1) preparing a first non-aqueous phase comprising a hydrophobic colorant at a temperature above its melting point;
(2) cooling down the first non-aqueous phase under the melting point temperature;
(3) preparing an aqueous phase and a second non-aqueous phase being substantially free of a hydrophobic colorant;
(4) mixing the aqueous phase and the second non-aqueous phase to obtain an oil-in-water emulsion;
(5) cooling down the oil-in-water emulsion under the melting point temperature of the first non-aqueous phase;
(6) mixing the oil-in-water emulsion and the first non-aqueous phase at a temperature below the melting point temperature.

According to a third aspect, the present invention relates to the use of the composition, according to the first aspect, to provide coloration onto the skin.

As used herein, the term "non-aqueous phase" may mean lipophilic phase, hydrophobic phase, and/or oily phase.
As used herein, the term "colorant" means a component suitable for providing coloration to the composition comprising it and/or for providing coloration to the skin when applied onto it.
As used herein, the term "hydrophobic colorant" means a colorant being inherently hydrophobic or a colorant being surface-modified and/or coated with a hydrophobic component. The same definition applies for "hydrophobic metal oxide pigment", "hydrophobic iron oxide pigment", "hydrophobic titanium dioxide pigment", and "hydrophobic mica".
As used herein, the term "hydrophilic colorant" means a colorant being inherently hydrophilic or a colorant being surface-modified and/or coated with a hydrophilic component. The same definition applies for "hydrophilic metal oxide pigment", "hydrophilic iron oxide pigment", "hydrophilic titanium dioxide pigment", and "hydrophilic mica".
As used herein, the term "dispersed phase" means that the phase is dispersed and suspended homogeneously, in the form of droplets, throughout a continuous phase. A dispersed phase may also be called a discrete or an internal phase while a continuous phase may also be called an external phase.
As used herein, the term "a composition (or a phase) being substantially free of a component" means that the composition (or the phase) comprises less than 0.1%, preferably 0%, of the component, by weight of the total composition (or by weight of the total phase).

As used herein, the term "a first phase and a second phase are distinct to each other" means that the first and the second phases are distinguishable from each other when the composition is observed under transmission microscopy at x100 objective lens.
As used herein, the term "room temperature" means about 25°C.
As used herein, the term "melting point" means the temperature at which the transition stage between a fully crystalline or partially crystalline solid state and a liquid state occurs.
As used herein, the term "stability over a sufficient period of time" means that the composition does not exhibit visible phase separation and/or droplet coalescence for at least 90 days, at room temperature, by direct visual observation.
As used herein, the term "satisfactory coloration over a sufficient period of time" means that the coloration does not fade for at least 90 days, at room temperature, under direct visual observation. This means that no change of color is noticeable for at least 90 days, at room temperature, by direct visual observation.
As used herein, the term "non-compatible components", means components which, when mixed altogether into a composition, exhibit an accelerated degradation; exhibit a diminution or a loss of activity; produce undesirable by-products; alter the aesthetics, the stability or the safety of the composition; and/or, exhibit a physical incompatibility.
As used herein, the term "C12 to C22 fatty acid (or fatty alcohol)" means that the fatty acid (or the fatty alcohol) comprises from 12 to 22 carbon atoms.
As used herein, the term "colorations being different from each other" means that these colorations, which may be quantified with LCH values to determine dE, are distinguishable by the human eye, when compared by direct visual observation.

### DETAILED DESCRIPTION OF THE INVENTION

According to a first aspect, the present invention relates to a cosmetic composition comprising a continuous aqueous phase, a first dispersed, non-aqueous phase and a second dispersed, non-aqueous phase.
The composition may comprise from 0.01% to 25%, preferably 0.01% to 10%, more preferably 0.1% to 5%, of the first non-aqueous phase, by weight of the total composition.
The composition may comprise from 0.1% to 50%, preferably 1% to 30%, more preferably 2% to 20%, of the second non-aqueous phase, by weight of the total composition.

The composition, according to the present invention, comprises a first dispersed, non-aqueous phase comprising a hydrophobic colorant. This phase is dispersed and suspended homogeneously, in the form of droplets, into the continuous aqueous phase.
This phase is solid at room temperature. As used herein, the term "solid" means that this phase exhibits a hardness from 1.0 N, preferably from 2.5 to 5 N. The solid phase exhibits a crystal organization.
This phase has a melting point from 27°C to 60°C. When the phase has a temperature below its melting point, this phase is solid and exhibits a crystal organization. By exhibiting a crystal organization, the multiple droplets forming this first phase do not tend to aggregate to each other and/or they do not tend to mix up with the second non-aqueous phase being substantially free of hydrophobic colorant. By increasing the temperature above the melting point, the state of the multiple droplets forming this first phase changes from solid to liquid and then these droplets would tend to mix up with the second dispersed, non-aqueous phase. The melting point can be reached, for example, when the composition is applied and then rubbed onto skin.

The first non-aqueous phase comprises a hydrophobic colorant. Depending on the hydrophobic colorant used, this phase may exhibit different coloration. Preferably, the hydrophobic colorant is selected from hydrophobic inorganic colorants. More preferably, the hydrophobic colorant is selected from hydrophobic iron oxide pigments, hydrophobic titanium dioxide pigments, hydrophobic micas or mixtures thereof. Still more preferably, the hydrophobic colorant is selected from hydrophobic iron oxide pigments, or mixtures thereof.
The hydrophobic iron oxide pigments may be selected from yellow hydrophobic iron oxide pigments, red hydrophobic iron oxide pigments, black hydrophobic iron oxide pigments or mixture thereof. When it is used a mixture of yellow, red and black hydrophobic iron oxide pigments, the first phase exhibits a dark coloration, i.e. from brown to black. Yellow iron oxide pigment is also known as goethite, ferric oxide hydrate or CI 77492. Red iron oxide pigment is also known as haematite, ferric oxide and CI 77491. Black iron oxide pigment is also known as magnetite, ferrous-ferric oxide and CI 77499. Examples of commercially available hydrophobic iron oxide pigments include FA50EYSI, FA55ERSI and FA60EBSI from Kobo products.
The hydrophobic titanium dioxide pigments may be selected from hydrophobic rutile titanium dioxide pigments, hydrophobic anastase titanium dioxide pigments, or mixture thereof. When a hydrophobic titanium dioxide pigment is used solely, the first phase exhibits a white coloration. When both hydrophobic titanium dioxide pigments and hydrophobic iron oxide pigments are used, the first phase may exhibit a white to light brown coloration. Examples of commercially available hydrophobic titanium dioxide pigments include FA65UMLO, FA65USI from Kobo Products Inc; and, MPY18S from Sensient.
The hydrophobic micas may be used alone or in combination with metal oxide pigments, including iron oxide pigments and titanium oxide pigments. When it is used hydrophobic micas, the first phase exhibits an iridescent coloration. Examples of commercially available hydrophobic micas include KTZ Bronze Dor-12 and KTZ Xian Vistas-11S2 from Kobo Products Inc.
The composition may comprise from 1% to 90%, preferably 5% to 80%, more preferably 10% to 70%, of a hydrophobic colorant, by weight of the total first non-aqueous phase. In addition, the composition may comprise from 0.001% to 10%, preferably 0.01% to 5%, more preferably 0.1% to 2%, of a hydrophobic colorant, by weight of the total composition.

The inventors have surprisingly found that a composition, into which the hydrophobic colorant is incorporated into a non-aqueous phase being solid at room temperature, exhibits satisfactory stability and aesthetics. Without wishing to be bound by any theory, it is believed that the crystal organisation prevents, or at least limits, the migration of the hydrophobic colorants into the continuous aqueous phase and/or into the second non-aqueous phase. It is also believed that the prevention of the migration of these colorants into other phases prevents them for reacting with other components present in the other phases and for impairing the stability of the composition.

The first non-aqueous phase are in the form of dispersed particles having preferably an average particle size from 10 µm, more preferably from 20 µm to 1000 µm, still more preferably from 20 µm to 500 µm, most preferably from 20 µm to 200 µm. The average particle size may be measured by optical microscopy using an image software, such as Image Pro-Plus v6.2 from Media Cybernetics.
It is advantageous having dispersed particles having an average particle size from 10 µm as these particles may be distinguished from the other phases by direct visual observation. This is advantageous to provide a composition having superior aesthetics, particularly when the droplets forming the first non-aqueous phase have a colouration and/or an appearance which is different from the coloration and/or the appearance of the other phases, as the first non-aqueous phase is visually distinguishable from the other phases.

The first non-aqueous phase may further comprise a lipophilic solidifying component. This solidifying component may be selected from waxes, fatty acids, fatty alcohols, or mixtures thereof.
The wax may be selected from natural or synthetic waxes. These waxes may be selected from candelilla waxes, carnauba waxes, beeswaxes, spermaceti, montan waxes, ozokerite waxes, ceresin waxes, paraffin waxes, silicone waxes, microcrystalline waxes, or mixtures thereof.
Silicone waxes may be selected from
- alkyloxy trimethylsilanes;
- alkylmethyl-dimethylsiloxanes having the formula Me₃SiO-[Si(Me)R-O-]ₓ-[SiMe₂-O-]_{y}-SiMe₃
wherein Me is a methyl group an R is an alkyl group, preferably an alkyl group with 8 to 22 carbon atoms, and x and y are whole number greater than 0 and independent to each other;
- or mixtures thereof.
   Alkyloxy trimethylsilane is preferably stearoxy trimethylsilane. An example of commercially available stearoxy trimethylsilane includes Dow Corning 580 Wax from Dow Corning Corporation. Alkylmethyl-dimethylsiloxane is preferably stearylmethyl-dimethylsiloxane, also called stearyl dimethicone. Examples of commercially available stearyl dimethicone include Dow Corning 2503 Cosmetic Wax from Dow Corning Corporation, and Abil Wax 9800 from Degussa Care & Surface Specialties.
   The fatty acids are preferably C12 to C22 fatty acids, more preferably stearic acid. An example of commercially available stearic acid includes Edenor L2SM from Cognis Corporation.
   The fatty alcohols are preferably C12 to C22 fatty acid alcohols, and more preferably stearyl alcohol. An example of commercially available stearyl alcohol includes Lanette 18 from Cognis Corporation or Dow Corning 580 Wax from Dow Corning Corporation (DC580 Wax material also comprising stearoxy trimethylsilane).
   The composition may comprise from 1% to 50%, preferably from 5% to 40%, more preferably 10% to 30%, of a lipophilic solidifying component, by weight of the total first non-aqueous phase. In addition, the composition may comprise from 0.001% to 10%, preferably 0.01% to 3%, more preferably 0.1% to 1%, of a solidifying component, by weight of the total composition.
   The type and the proportion of the lipophilic solidifying components used would determine the melting point of the first non-aqueous composition.

The first non-aqueous phase may comprise a weight (by weight of the total first non-aqueous) ratio of lipophilic solidifying component : hydrophobic colorant from 10:1 to 1:10, preferably from 5:1 to 1:5, more preferably from 1:1 to 1:3.

The composition, according to the present invention, comprises a second, dispersed non-aqueous phase. This phase is dispersed and suspended homogeneously, in the form of droplets, into the continuous aqueous phase. The phase is distinct from the first non-aqueous phase. This phase is preferably substantially free of hydrophobic colorant (as defined above). This phase is preferably an oily phase.
This phase is in the form of droplets having an average particle size of 10 µm or less. The average particle size is measured by optical microscopy as detailed herein.
The composition may comprise from 0.1% to 50%, preferably from 1 to 30%, more preferably from 2% to 20%, of the second non-aqueous phase, by weight of the total composition.

The second non-aqueous phase may comprise a lipophilic component, which component is non-compatible with hydrophobic colorants. Preferably, this lipophilic component is selected from dibenzoylmethane derivatives. More preferably, this lipophilic component is butylmethoxy-dibenzoylmethane. Examples of commercially available butylmethoxy-dibenzoylmethane, also called avobenzone, include Eusolex 9020 from EMD Chemicals Inc., Neo heliopan 357 from Symrice and Parsol 1789 from DSM Nutritional Products, Inc.
The composition may comprise from 0.01% to 10%, preferably from 0.1% to 5%, of a lipophilic component being non-compatible with hydrophobic colorants, by weight of the total composition.
Hydrophobic metal oxide pigments are known to be non-compatible with dibenzoylmethane derivatives. When both hydrophobic colorants and dibenzoylmethane derivatives are incorporated altogether into a composition, this composition tends to be unstable after 90 days at room temperature. Particularly, dibenzoylmethane derivatives tend to be degraded into by-products, such degradation resulting in an altered aesthetics with pinkish/reddish coloration. In contrast, the inventors have surprisingly found that the incorporation of hydrophobic metal oxide pigments and dibenzoylmethane derivative into two different dispersed, non-aqueous phases obviates the alteration of the aesthetics and/or the stability of the composition. Without wishing to be bound by any theory, it is believed that the solid state of first non-aqueous phase at room temperature, prevents the migration of hydrophobic metal oxide pigments into the other phases and prevents, therefore, the reaction between hydrophobic metal oxide pigments and dibenzoylmethane derivatives.

The second non-aqueous phase being substantially free of hydrophobic colorant may comprise an oil. This oil may be selected from volatile oils, non-volatile oils or mixtures thereof. As used herein, the term "non-volatile" when employed in relation to an oil includes oils that fulfill at least one of the following definitions: (a) the oil exhibits a vapor pressure of no more than 0.2mm Hg at 25°C and one atmosphere pressure; (b) the oil has a boiling point at one atmosphere of at least 300°C. As used herein, the term "volatile" when employed in relation to oils includes materials that are not "non-volatile" as previously defined herein.
This composition may comprise from 0.1% to 10%, preferably from 1% to 5%, oil by weight of the total composition.
Non-volatile oils may be selected from non-volatile silicone oils, non-volatile hydrocarbon oils and mixtures thereof. Suitable non-volatile silicone oils include linear polymethylsiloxanes and, preferably, non-volatile silicone oils are high molecular weight dimethicones. Examples of commercially available linear polymethylsiloxanes include DC 200 Fluid 20Cst, DC 200 Fluid 100Cst, DC 200 Fluid 350Cst from Dow Corning Corporation.
Suitable non-volatile hydrocarbon oils may be selected from fatty acid esters, branched esters of diglycerin or triglycerin, the esters or 1,2,3,4 butane triol or erythritol, di erythritol or tri erthyritol, or mixtures thereof. Preferably, non-volatile hydrocarbon oils comprise isopropyl palmitate, erythrityl triethylhexanoate (available as Salacos E-38 from Nisshin Oilio), polyglyceryl-2 triisostearate (available as Cosmol 43V from Nisshin Oilio), diethyl hexyl carbonate (available as Tegosoft DEC from Degussa), dicapryl ether (available as Cetiol OE from Cognis AG), dicapryl carbonate (available as Cetiol CC from Cognis AG), isononyl isononanoate (available as Lanol 99 from Seppic), tridecyl neopentanoate (supplied as Ceraphyl 55 from International Speciality Products), or mixture thereof.
Volatile oils may be selected from volatile silicone oils, both functionalised and non-functionalised, volatile hydrocarbon oils and mixtures thereof. Volatile oil useful in the present invention may exhibit one or more of the following characteristics - it may be saturated or unsaturated, have a straight or branched chain or a cyclic structure.

Examples of volatile hydrocarbons oils include polydecanes such as isododecane and isodecane (e.g., Permethyl-99A which is available from Presperse Inc.) and the C₇-C₁₅ isoparaffins (such as the Isopar Series available from Exxon Chemicals).

The volatile silicone oil may be selected from volatile cyclic silicone oils corresponding to the formula: 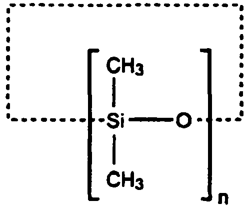 wherein n is from 3 to 7,
volatile linear silicone oils corresponding to the formula

(CH₃)₃Si-O-[Si(CH₃)₂-O]ₘ-si(CH₃)₃

wherein m is from 1 to 20 preferably from 3 to 12, or mixture thereof.

Preferably, the cyclic volatile silicone oil is selected cyclopentasiloxane, cyclohexasiloxane or mixture thereof. Examples of commercially available volatile cyclic silicone oils include DC 244, DC 245, DC 344, and DC 345 from Dow Coming Corp.; SF-1204 and SF-1202 Silicone Fluids from Momentive Performance Materials; GE 7207 and 7158 from General Electric Co.); and, SWS-03314 from SWS Silicones Corp.

Preferably, the linear volatile silicone oil is a linear polymethylsiloxane. Example of commercially available linear polymethylsiloxanes includes DC 200 Fluid, 5Cst from Dow Corning Corp.

The composition, according to the present invention, comprises a continuous aqueous phase. The composition may comprise from 10% to 95%, preferably 20% to 80%, more preferably 40% to 80%, of a continuous aqueous phase, by weight of the total composition.

The aqueous phase may comprise a hydrophilic colorant. Preferably, the hydrophilic colorant is selected from hydrophilic inorganic colorants. More preferably, the hydrophilic colorant is selected from hydrophilic iron oxide pigments, hydrophilic titanium dioxide pigments, hydrophilic micas, or mixtures thereof.

Hydrophilic iron oxide pigments may be selected from yellow hydrophilic iron oxide pigments, red hydrophilic iron oxide pigments, black hydrophilic iron oxide pigments or mixtures thereof. Examples of commercially available hydrophilic titanium dioxide pigments include GLW45GYAP, GLW55GRAP, GLW60GBAP from Kobo Products Inc. Hydrophilic titanium dioxide pigments may be selected from hydrophilic rutile titanium dioxide pigments, hydrophilic anastase titanium dioxide pigment, or mixtures thereof. Examples of commercially available hydrophilic titanium dioxide pigments include GLW75PFAP, GLW65KTAP from Kobo Products Inc. Hydrophilic micas may be used alone or in combination with metal oxide pigments, including iron oxide pigments and titanium oxide pigments. Examples of commercially available hydrophilic micas include Prestige Bright Bronze and Prestige Bright Sungold from Eckart GmbH.

Preferably, the composition comprises aqueous phase comprises a hydrophilic colorant, which hydrophilic colorant being different from the hydrophobic colorant present into the first non-aqueous phase. Most preferably, the composition comprises an aqueous phase comprising hydrophilic titanium dioxide pigments and a first non-aqueous phase comprising hydrophobic iron oxide pigments. Alternatively, the composition comprises an aqueous phase comprising hydrophilic iron oxide pigments and a first non-aqueous phase comprising hydrophobic titanium dioxide pigments.
The composition may comprise from 0.001% to 10%, preferably from 0.01% to 3%, more preferably from 0.1% to 1%, of a hydrophilic colorant, by weight of the total composition.

The composition may comprise a first non-aqueous phase having a coloration being different from the coloration of the continuous aqueous phase. The difference may be assessed by direct visual observation.
Preferably, the composition comprises an aqueous phase having a light coloration and a first non-aqueous phase having a dark coloration. Alternatively, the composition comprises an aqueous phase having a dark coloration and a first non-aqueous phase having a light coloration. As used herein, the term "light coloration" means a white to beige coloration. As used herein, the term "dark coloration" means a brown to black coloration.
The difference of coloration may be achieved by having the aqueous phase and the first non-aqueous phase comprising different types of colorants. For example, the aqueous phase comprising hydrophilic titanium dioxide pigments has a light coloration while the aqueous phase comprising hydrophilic iron oxide pigments has a dark coloration. Likewise, the first non-aqueous phase comprising hydrophobic titanium dioxide pigments has a light coloration while the first non-aqueous phase comprising hydrophobic iron oxide pigments has a dark coloration.

The composition may also comprise a first non-aqueous phase having a darker coloration than the continuous aqueous phase. Alternatively, the composition may also comprise a first non-aqueous phase having a lighter coloration than the continuous aqueous phase.
The difference of degree of coloration may be achieved by having the aqueous phase and the first non-aqueous phase comprising different proportions of the same type of colorant. For example, it may be obtained a first non-aqueous phase with a darker coloration than the continuous aqueous phase when this first non-aqueous phase comprises a higher proportion of hydrophobic iron oxide pigments than the proportion of hydrophilic iron oxide pigments present into the continuous aqueous phase.

Most preferably, when the composition comprises a first non-aqueous phase having a coloration being different from the coloration of the continuous aqueous phase, the first non-aqueous phase is in the form of dispersed particles having preferably an average particle size from 10 µm. It is advantageous having a first non-aqueous phase being distinguishable from the other phases by direct visual observation to provide a composition having superior aesthetics. Particularly, it is advantageous having a composition exhibiting a bi-colored appearance.

The composition may further comprise a surfactant. Generally, the surfactant helps disperse and suspend the first and the second dispersed, non-aqueous phase within the continuous aqueous phase. Preferably, the surfactant is selected from nonionic surfactants, cationic surfactants, anionic surfactants, zwitterionic surfactants, amphoteric surfactants, or mixtures thereof. More preferably, the surfactant is selected from nonionic surfactants. Suitable surfactants may be found in McCutcheon's, Detergents and Emulsifiers, North American Edition (1986), published by Allured Publishing Corporation. Examples of commercially available surfactants include Myrj 59 (PEG-100 stearate) from Uniquema Americas; Arlacel 60 (Sorbitan Stearate) from Uniquema Americas, and Emulgade PL 68/50 (cetearyl glucoside and cetearyl alcohol) from Cognis Deuschland GmbH & CO. KG. The composition may comprise from 0.05% to 10%, preferably from 1% to 6%, and more preferably from 1% to 3% of a surfactant by weight of the total composition.

The composition may further comprise a thickener. The thickener may be selected from C₁₂-C₂₄ fatty acids; C₁₀-C₃₀ fatty alcohols; N-fatty glutamic acid dialylamides; carboxy methyl cellulose or derivatives thereof; polysaccharide gums, such as xanthan gum and guar gum; starch or derivatives thereof; particulate thickeners, such as hydrated colloidal aluminium silicate and magnesium aluminium silicate; carboxyvinyl polymers; sodium acrylate copolymers and hydrophobically modified sodium acrylate copolymers; polyacrylamide copolymers; polyacrylates, such as polyacrylate-13; acrylamide/ammonium acrylate copolymer; ammonium acryloyldimethyl taurate and ammonium acryloyldimethyl taurate crosspolymers and copolymers and hydrophobically modified ammonium acryloyldimethyl taurate copolymers; catinionically charged polymers, such as polyquaternium polymers and polyquaternium copolymers; or mixtures thereof. Examples of commercially available thickeners include Sepigel 305, Simulgel NS, Simulgel I-NS 100 from Seppic Inc.; Luvigel EM from BASF Corporation. The composition may comprise from 0.01% to 10%, preferably from 0.1% to 7% and more preferably from 1% to 3% thickener by weight of the total composition.

The composition may further comprise an additional hydrophobic organic sunscreen component. As used herein, "additional hydrophobic organic sunscreen component" means a hydrophobic organic sunscreen component other than dibenzoylmethane derivatives. When present, the hydrophobic organic sunscreen component is selected from benzophenone derivatives; cinnamic derivatives; salicylic derivatives; camphor derivatives; triazine derivatives; anthranilate derivatives; p-aminobenzoic acid derivatives; alkyl β,β-diphenylacrylate derivatives; α-cyano β,β-diphenylacrylate derivatives; or mixtures thereof. The cosmetic composition may comprise from 0.01% to 15%, preferably from 0.1% to 10%, and more preferably from 1% to 8% of an additional hydrophobic organic sunscreen component, by weight of the total composition.

The composition may further comprise a hydrophilic organic sunscreen component. The hydrophilic organic sunscreen component is preferably 2-phenylbenzimidazole-5-sulfonic acid (PBSA). The cosmetic composition may comprise from 0.1% to 5%, preferably from 0.5% to 2.25%, and more preferably from 0.75% to 1.5% of a hydrophilic organic sunscreen component, by weight of the total composition.

The composition may further comprise a humectant. The humectant is preferably selected from polyhydric alcohols; more preferably from glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof. Still more preferably, the humectant is glycerin. The cosmetic composition according to the invention may comprise from 0.1 to 30%, preferably from 5% to 25% and more preferably from 7% to 15% humectant by weight of the total composition.

The composition may also comprise a hydrophilic vitamin component. The hydrophilic vitamin component may be selected from vitamin B₃, salts or esters thereof; pro-vitamin B₅, salts or esters thereof; vitamin C, salts or thereof; hydrophilic derivatives of vitamin E, such as tetramethylchromanol glucosides and 6-hydroxy-2,5,7,8-tetramethylchroman-2-caroxylic acid; or mixtures thereof. The hydrophilic vitamin component is preferably selected from vitamin B₃, salts or esters thereof; vitamin B₅, salts or esters thereof; or mixtures thereof. More preferably, the hydrophilic vitamin component comprises vitamin B₃, salts or esters thereof, or mixtures thereof; e.g. niacinamide. The composition may comprise from 0.01% to 10%, preferably from 0.1% to 9%, more preferably from 0.5% to 5% of hydrophilic vitamin component by weight of the total composition.

The composition may further comprise a hydrophobic vitamin component. The hydrophobic vitamin component may be selected from vitamin E or hydrophobic derivatives thereof; vitamin D or derivatives thereof; or mixtures thereof. The composition may comprise from 0.01% to 10%, preferably from 0.1% to 9%, more preferably from 0.5% to 5% of a hydrophobic vitamin component by weight of the total composition.

The composition may further comprise a tanning active. Preferably, the tanning active is selected from dihydroxyacetone (DHA), salts, derivatives or tautomers thereof, or mixtures thereof. The composition may comprise from 0.01% to 10%, preferably from 0.05% to 5%, and more preferably from 0.1% to 1% of a tanning active by weight of the total composition.

The composition may further comprise at least one sugar amine. This sugar amine may be synthetic or natural in origin and can be used as pure compounds or mixtures of compounds (e.g., extracts from natural sources or mixtures of synthetic materials). Preferably, sugar amine is selected from glucosamine, N-acetyl glucosamine, mannosamine, N-acetyl mannosamine, galactosamine, N-acetyl galactosamine, isomers thereof (e.g., stereoisomers), salts thereof (e.g., HCl salt), or mixtures thereof. The composition may comprise from 0.01% to 15%, preferably from 0.1% to 10%, and more preferably from 0.5% to 5% of a sugar amine by weight of the total composition.

The composition may further comprise a hexaminidine compound, salts or derivatives thereof, or mixtures thereof. Preferably, hexaminidine compound comprises compounds corresponding to the following chemical structure: 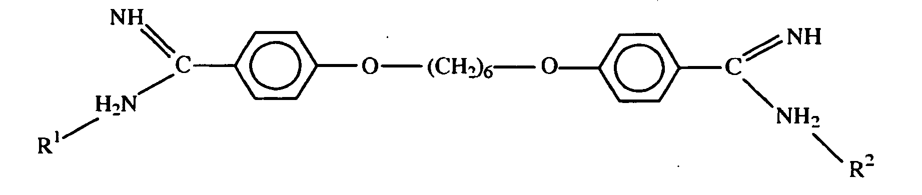 wherein R¹ and R² are organic acids (e.g., sulfonic acids, etc.). The composition may comprise from 0.001% to 10%, preferably from 0.01% to 5%, and more preferably from 0.02% to 2.5% of a hexaminidine compound by weight of the total composition.

A variety of additional optional ingredients may be incorporated into the compositions of the present invention. Non-limiting examples of these additional ingredients include additional skin care actives such as bisabolol, dialkanoyl hydroxyproline compounds, farnesol, flavonoids, guanidine (e.g., amino guanidine), N-acyl amino acid compounds, peptides (e.g. Matrixyl [Pal-KTTKS]), phytantriol, phytosterols, salicylic compounds, urea as well as compounds such as anti-acne compounds (e.g. resorcinol, erythromycin); antioxidants compounds (e.g., phytosterols, lipoic acid); skin soothing and healing agents; anti-wrinkle/anti-atrophy actives; conditioning agents; anti-inflammatory agents; skin lightening agents; antimicrobial/antibacterial/antifungal actives; chelators and sequestrants; and agents suitable for aesthetic purposes such as essential oils, fragrances, skin sensates, opacifiers, aromatic compounds (e.g., clove oil, menthol, camphor, eucalyptus oil, and eugenol), preservatives, or mixtures thereof.

According to the second aspect, the present invention relates to a process for preparing a cosmetic composition, according to the first aspect, this process comprising the steps of:
(1) preparing a first non-aqueous phase comprising a hydrophobic colorant at a temperature above its melting point;
(2) cooling down the first non-aqueous phase under the melting point temperature;
(3) preparing an aqueous phase and a second non-aqueous phase being substantially free of a hydrophobic colorant;
(4) mixing the aqueous phase and the second non-aqueous phase to obtain an oil-in-water emulsion;
(5) cooling down the oil-in-water emulsion under the melting point temperature of the first non-aqueous phase;
(6) mixing the oil-in-water emulsion and the first non-aqueous phase at a temperature below the melting point temperature.

Steps (3) to (5) may be carried out before or simultaneously to steps (1) and (2).

The step of mixing the oil-in-water emulsion and the first non-aqueous phase may be conducted under shear rate. The shear rate applied may impact on the average particle size of the first non-aqueous phase. Usually, the average particle size would be inversely proportional to the shear rate applied to the composition.

This process is advantageous as it improves the stability of the composition and it prevents the mixing of the first dispersed, non-aqueous phase with the second dispersed, non-aqueous phase.

According to a third aspect, the present invention relates to the use of the composition, according to the first aspect, to provide coloration onto the skin.
This composition is particularly advantageous to provide coloration to the skin as, after application onto skin, the first dispersed, non-aqueous phase, in the form of droplets, tends to be liquefied under the rubbing effect. The liquefaction of these droplets allows a homogeneous application of the hydrophobic metal oxide pigments onto the skin and allows providing an even coloration.

### Methodology

Measurement of the hardness of the first non-aqueous phase comprising a hydrophobic colorant

The hardness of the first non-aqueous phase may be measured using Force gauges. See for example the Chatillon® TCD200 Series digital test stand from Chatillon, used with a Chatillon® Force Gauge DFGS 10 equipped with rod extension and a 5 mm-diameter, 60-degree cone gauge tip.
50g of a first non-aqueous phase is prepared at a temperature above the melting point, it is poured into a 10mL Pyrex® beaker and it is cooled down at room temperature for 60 min. When the first phase is solid, this phase is placed under the gauge tip and the gauge tip is lowered until it touches the upper surface. This defines the zero deflection point. Then, the gauge is lowered at a speed of 10 mm/min at room temperature until it reaches a deflection of 15 mm. The peak compression is recorded and corresponds to the average force (or hardness) expressed in Newtons (N).
Measurement of the melting point by thermal analysis

The melting point of the first dispersed non-aqueous phase may be measured by differential scanning calorimetry (DSC) using a Differential scanning Calorimeter, such as the calorimeter DSC Q2000 provided by TA Instruments. The determination of temperature of melting is conducted according to the international standard ISO 11357-3:1999/Amd.1:2005(E). The melting point, or the temperature at which the transition stage occurs, is characterized by the most endothermic peak in the DSC curve.
Measurement of the average particle size of the droplets of the first dispersed, non-aqueous phase

The average particle size of the first non-aqueous phase may be measured by a sizing and image-analysis software, e.g. Image Pro-Plus v6.2 from Media Cybernetics.
For example, the first non-aqueous phase, in the form of droplets, comprising hydrophobic iron oxide pigments exhibit a dark coloration. When wanting to measure the average particle size of these droplets, the software is configured to select automatically dark objects and running the software. The average (mean) particle size, as measured using Image Pro-Plus v6.2, is the average length of diameters measured at 2 degree intervals and passing through the objects centroid.
Measurement of the coloration of the phases and/or the composition

The coloration of a phase or a composition may be assessed by using the LCH color measurement using Microflash 200d Part Number 1200-0743n from Datacolor International. As used herein, the term "LCH" means L for Ligthness, C for Brightness and H for the actual color. The color difference of two compositions or phases can be evaluated via the dE value. dE can be measured according to the CMC method developed by the Colour Measurement Committee of the Society of Dyes and Colourists of Great Britain (see British Standards BS:6923 "Method for calculation of small colour differences").

### Example

The following examples further describe and demonstrate the preferred embodiments within the scope of the present invention. The examples are given solely for the purpose of illustration, and are not to be construed as limitations of the present invention since many variations thereof are possible without departing from its scope.
All weights provided in these examples are weights of the commercially available materials, including active(s) and/or solvent(s) and/or by-product(s).

### Examples 1 to 6

The first non-aqueous phase is prepared by mixing DC580 or DC2503 with the hydrophobic colorants, at a temperature above its melting point, typically above 60°C, until an homogeneous phase is obtained. This first non-aqueous phase is then cooled down below the melting point, typically at room temperature.
The second non-aqueous phase is prepared under stirring and at a temperature ranging from 50°C to 75°C. The phase is stirred till the components are fully dissolved. The aqueous phase is prepared under stirring and at a temperature ranging from 50°C to 75°C. The second non-aqueous phase and the aqueous phase are mixed altogether at 75°C and at 13,500 rpm for few minutes to obtain an oil-in-water emulsion. The emulsion obtained is then cooled down at room temperature.
The first non-aqueous phase is then added into the water-in-oil emulsion at room temperature. The composition obtained is stirred for 10 minutes at 30 min⁻¹.

| Ingredients | Proportions (wt %/wt %) |
|---|---|
| | 1 |
| Avobenzone | 2.0 |
| Behenyl alcohol | 1.0 |
| Cetearyl glucoside; cetearyl alcohol ¹ | 0.1 |
| Cetyl alcohol | 0.5 |
| Fragrance | 0.1 |
| Glycerin | 7.0 |
| Hydrophilic mica ² | 1.0 |
| Hydrophilic mica ³ | 1.0 |
| Hydrophilic titanium dioxide ⁴ | 2.0 |
| Hydrophilic iron oxide ⁵ | --- |
| Isopropyl palmitate | 1.0 |
| Niacinamide | 1.0 |
| Octocrylene | 1.0 |
| Octyl salicylate | 4.0 |
| Panthenol | 1 |
| PBSA | 1.0 |
| PEG-100 stearate | 0.1 |
| Preservatives | 0.5 |
| Silicone | 1.0 |
| Sorbitan stearate | 1 |
| Stearic acid | 0.1 |
| Stearyl alcohol 95% | 1 |
| Thickener ⁶ | 2.0 |
| Tocopherol acetate | 0.5 |
| Triethanolamine | 0.75 |
| Water | Qsp |
| Hydrophobic iron oxides ⁷ | 1.5 |
| Hydrophobic titanium dioxide ⁸ | --- |
| Hydrophobic mica ⁹ | --- |
| DC580 | 0.4 |
| DC2503 | --- |

| | |
|---|---|
| 1: Emulgade from Cognis; 2: KTZ Interval Gold from Kobo Products Inc; 3: Prestige Bright Orange from Eckart GmbH; 4: GLW65KTAP from Kobo Products Inc; 5: GLW45GYAP, GLW55GRAP and GLW60GBAP from Kobo Products Inc; 6: Simulgel INS 100 and/or Sepigel 305 from Seppic; 7: FA50EYSI, FA55ERSI and FA60EBSI from Kobo Products Inc; 8: FA65USI from Kobo Products Inc; 9: KTZ Xian Vista - 11S2 from Kobo Products Inc. | |

In the composition according to example 1, the first non-aqueous phase exhibits a dark coloration while the continuous phase exhibits a light coloration.
As the first non-aqueous phase is in the form of droplets having an average particle size from about 20 µm to about 200 µm, this first non-aqueous phase is distinguishable from the continuous aqueous phase by direct visual observation. This gives a composition having superior aesthetics.
In addition, the first non-aqueous phase has a melting point of about 47°C. When stored at room temperature (e.g. on the shelf of a store), this first non-aqueous phase is solid. When the composition is applied onto the skin, the first non-aqueous phase is liquefied under the rubbing action. This allows the hydrophobic colorants to be spread homogeneously over the skin to confer a coloration to the skin.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A cosmetic composition comprising:
(1) a continuous aqueous phase;
(2) a first dispersed, non-aqueous phase being solid at room temperature, having a melting point from 27°C to 60°C, and comprising a hydrophobic colorant; and,
(3) a second dispersed, non-aqueous phase being distinct from the first non-aqueous phase, and being substantially free of a hydrophobic colorant,
wherein the second non-aqueous phase comprises at least one lipophilic component, which component being non-compatible with hydrophobic colorants.

2. A composition, according to claim 1, wherein the first non-aqueous phase is in the form of dispersed droplets having an average particle size from 10 µm.

3. A composition, according to any of the preceding claims, wherein the first non-aqueous phase comprises from 1% to 50% of a lipophilic solidifying component, by weight of the total first non-aqueous phase.

4. A composition, according to claim 3, wherein the lipophilic solidifying component is selected from waxes, fatty acids, fatty alcohols, or mixtures thereof.

5. A composition, according to any of the preceding claims, wherein the first non-aqueous phase comprises from 1 to 90% of a hydrophobic colorant, by weight of the total first non-aqueous phase.

6. A composition, according to any of the preceding claims, wherein the hydrophobic colorant is selected from hydrophobic iron oxide pigments, hydrophobic titanium dioxide pigments, hydrophobic micas, or mixtures thereof, preferably the hydrophobic metal oxide pigment is selected from hydrophobic iron oxide pigments or mixtures thereof.

7. A composition, according to any preceding claim, wherein said lipophilic component is a dibenzoylmethane derivative, preferably butylmethoxy-dibenzoylmethane.

8. A composition, according to any of the preceding claims, wherein the aqueous phase comprises a hydrophilic colorant, which hydrophilic colorant being different from the hydrophobic colorant.

9. A composition, according to any of the preceding claims, wherein the first non-aqueous phase has a coloration being different from the coloration of the aqueous phase.

10. Process for preparing a cosmetic composition, as defined in claims 1 to 9, this process comprising the steps of:
(1) preparing a first non-aqueous phase comprising a hydrophobic colorant at a temperature above its melting point;
(2) cooling down the first non-aqueous phase under the melting point temperature;
(3) preparing an aqueous phase and a second non-aqueous phase being substantially free of a hydrophobic colorant;
(4) mixing the aqueous phase and the second non-aqueous phase to obtain an oil-in-water emulsion;
(5) cooling down the oil-in-water emulsion under the melting point temperature of the first non-aqueous phase;
(6) mixing the oil-in-water emulsion and the first non-aqueous phase at a temperature below the melting point temperature.

11. Use of the composition, as defined in claims 1 to 9, to provide coloration onto the skin.

## Patentansprüche

1. Kosmetikzusammensetzung, umfassend:
(1) eine wässrige Dispersionsphase,
(2) eine erste dispergierte, nichtwässrige Phase, die bei Raumtemperatur fest ist, einen Schmelzpunkt von 27 °C bis 60 °C aufweist und einen hydrophoben Farbstoff umfasst, und
(3) eine zweite dispergierte, nichtwässrige Phase, die sich von der ersten nichtwässrigen Phase unterscheidet und im Wesentlichen frei von einem hydrophoben Farbstoff ist,
wobei die zweite nichtwässrige Phase mindestens einen lipophilen Bestandteil umfasst, der nicht mit hydrophoben Farbstoffen kompatibel ist.

2. Zusammensetzung nach Anspruch 1, wobei die erste nichtwässrige Phase in Form von dispergierten Tröpfchen mit einer durchschnittlichen Teilchengröße ab 10 µm vorliegt.

3. Zusammensetzung nach einem der vorstehenden Ansprüche, wobei die erste nichtwässrige Phase zu 1 Gew.-% bis 50 Gew.-% der gesamten ersten nichtwässrigen Phase einen lipophilen Verfestigungsbestandteil umfasst.

4. Zusammensetzung nach Anspruch 3, wobei der lipophile Verfestigungsbestandteil ausgewählt ist aus Wachsen, Fettsäuren, Fettalkoholen oder Mischungen davon.

5. Zusammensetzung nach einem der vorstehenden Ansprüche, wobei die erste nichtwässrige Phase zu 1 bis 90 Gew.-% der gesamten ersten nichtwässrigen Phase einen hydrophoben Farbstoff umfasst.

6. Zusammensetzung nach einem der vorstehenden Ansprüche, wobei der hydrophobe Farbstoff ausgewählt ist aus hydrophoben Eisenoxidpigmenten, hydrophoben Titandioxidpigmenten, hydrophoben Glimmermineralen oder Mischungen davon, vorzugsweise ist das hydrophobe Metalloxidpigment ausgewählt aus hydrophoben Eisenoxidpigmenten oder Mischungen davon.

7. Zusammensetzung nach einem der vorstehenden Ansprüche, wobei der lipophile Bestandteil ein Dibenzoylmethan-Derivat, vorzugsweise Butylmethoxydibenzoylmethan ist.

8. Zusammensetzung nach einem der vorstehenden Ansprüche, wobei die wässrige Phase einen hydrophilen Farbstoff umfasst, der sich von dem hydrophoben Farbstoff unterscheidet.

9. Zusammensetzung nach einem der vorstehenden Ansprüche, wobei die erste nichtwässrige Phase eine Färbung aufweist, die sich von der Färbung der wässrigen Phase unterscheidet.

10. Verfahren zum Herstellen einer Kosmetikzusammensetzung, wie in Anspruch 1 bis 9 definiert, wobei dieses Verfahren die folgenden Schritte umfasst:
(1) Herstellen einer ersten nichtwässrigen Phase, umfassend einen hydrophoben Farbstoff, bei einer Temperatur oberhalb seines Schmelzpunkts,
(2) Abkühlen der ersten nichtwässrigen Phase unter die Schmelzpunkttemperatur,
(3) Herstellen einer wässrigen Phase und einer zweiten nichtwässrigen Phase, die im Wesentlichen frei von einem hydrophoben Farbstoff ist,
(4) Mischen der wässrigen Phase und der zweiten nichtwässrigen Phase, um eine Öl-in-Wasser-Emulsion zu erhalten,
(5) Abkühlen der Öl-in-Wasser-Emulsion unter die Schmelzpunkttemperatur der ersten nichtwässrigen Phase,
(6) Mischen der Öl-in-Wasser-Emulsion und der ersten nichtwässrigen Phase bei einer Temperatur unterhalb der Schmelzpunkttemperatur.

11. Verwendung der Zusammensetzung, wie in den Anspruch 1 bis 9 definiert, um der Haut eine Färbung zu verleihen.

## Revendications

1. Composition cosmétique comprenant :
(1) une phase aqueuse continue;
(2) une première phase non aqueuse dispersée qui est solide à la température ambiante, ayant un point de fusion allant de 27 °C à 60 °C, et comprenant un colorant hydrophobe; et,
(3) une deuxième phase non aqueuse dispersée distincte de la première phase non aqueuse, et qui est essentiellement dépourvue d'un colorant hydrophobe,
dans laquelle la deuxième phase non aqueuse comprend au moins un composant lipophile, lequel composant étant non compatible avec les colorants hydrophobes.

2. Composition selon la revendication 1, dans laquelle la première phase non aqueuse est sous la forme de gouttelettes dispersées ayant une taille moyenne des particules à partir de 10 µm.

3. Composition selon l'une quelconque des revendications précédentes, dans laquelle la première phase non aqueuse comprend de 1 % à 50 % d'un composant solidifiant lipophile, en poids de la première phase non aqueuse totale.

4. Composition selon la revendication 3, dans laquelle le composant solidifiant lipophile est choisi parmi des cires, des acides gras, des alcools gras, ou leurs mélanges.

5. Composition selon l'une quelconque des revendications précédentes, dans laquelle la première phase non aqueuse comprend de 1 % à 90 % d'un colorant hydrophobe, en poids de la première phase non aqueuse totale.

6. Composition selon l'une quelconque des revendications précédentes, dans laquelle le colorant hydrophobe est choisi parmi des pigments hydrophobes à l'oxyde de fer, des pigments hydrophobes au dioxyde de titane, des micas hydrophobes, ou leurs mélanges, de préférence le pigment hydrophobe à l'oxyde métallique est choisi parmi les pigments hydrophobes à l'oxyde de fer ou leurs mélanges.

7. Composition selon l'une quelconque des revendications précédentes, dans laquelle ledit composant lipophile est un dérivé de dibenzoylméthane, de préférence du butylméthoxy-dibenzoylméthane.

8. Composition selon l'une quelconque des revendications précédentes, dans laquelle la phase aqueuse comprend un colorant hydrophile, lequel colorant hydrophile étant différent du colorant hydrophobe.

9. Composition selon l'une quelconque des revendications précédentes, dans laquelle la première phase non aqueuse a une coloration qui est différente de la coloration de la phase aqueuse.

10. Procédé pour préparer une composition cosmétique, telle que définie dans les revendications 1 à 9, ce procédé comprenant les étapes consistant à :
(1) préparer une première phase non aqueuse comprenant un colorant hydrophobe à une température supérieure à son point de fusion ;
(2) refroidir la première phase non aqueuse sous la température du point de fusion;
(3) préparer une phase aqueuse et une deuxième phase non aqueuse essentiellement dépourvue d'un colorant hydrophobe;
(4) mélanger la phase aqueuse et la deuxième phase non aqueuse de façon à obtenir une émulsion huile-dans-eau ;
(5) refroidir l'émulsion huile-dans-eau sous la température du point de fusion de la première phase non aqueuse ;
(6) mélanger l'émulsion huile-dans-eau et la première phase non aqueuse à une température inférieure à la température du point de fusion.

11. Utilisation de la composition, telle que définie dans les revendications 1 à 9, pour fournir une coloration sur la peau.
